# EUROPÄISCHE PATENTSCHRIFT

(11) **EP 0 775 135 B1**
(45) Veröffentlichungstag und Bekanntmachung des Hinweises auf die Patenterteilung: **04.06.2003**
(21) Anmeldenummer: 95926970.5
(22) Anmeldetag: 21.07.1995
(51) Int. Cl.: C07D 403/06, A61K 31/40

(54) **N-SUBSTITUIERTE 3-AZABICYCLO(3.2.0)HEPTAN-DERIVATE ALS NEUROLEPTIKA**
N-SUBSTITUTED 3-AZABICYCLO (3.2.0)HEPTANE DERIVATIVES USEFUL AS NEUROLEPTICS
DERIVES DE 3-AZABICYCLO(3.2.0)HEPTANES A SUBSTITUTION AZOTE ET LEUR UTILISATION COMME NEUROLEPTIQUES

(30) Priorität: 04.08.1994 DE 4427648
(43) Veröffentlichungstag der Anmeldung: 28.05.1997
(73) Patentinhaber: Abbott GmbH & Co. KG, 65205 Wiesbaden (DE)
(72) Erfinder: STEINER, Gerd, D-67281 Kirchheim (DE); MUNSCHAUER, Rainer, D-67434 Neustadt (DE); HÖGER, Thomas, D-68535 Edingen-Neckarhausen (DE); UNGER, Liliane, D-67065 Ludwigshafen (DE); TESCHENDORF, Hans-Jürgen, D-67373 Dudenhofen (DE)
(74) Vertreter: Grünecker, Kinkeldey, Stockmair & Schwanhäusser Anwaltssozietät
(86) Internationale Anmeldenummer: EP9502893
(87) Internationale Veröffentlichungsnummer: WO96004272

(56) Entgegenhaltungen:
- WO-A-95/15312
- DE-A- 4 243 287

## Beschreibung

Die Erfindung betrifft neue N-substituierte Azabicycloheptan-Derivate, deren Herstellung und Verwendung zur Herstellung von Pharmawirkstoffen.

Es ist bekannt, daß N-substituierte Azabicycloheptan-Derivate überraschende Affinitäten zu Dopamin- und Serotonin-Rezeptor-Subtypen aufweisen (DE-A-42 43 287 Äquivalent zu WO-A-94 00 458 und DE-A-42 19 973 der WO-A-95 15 312 entspricht). Hierbei spielen die beobachteten hohen Affinitäten zum D₄-Dopaminrezeptor-Subtyp eine besondere Rolle.

Es wurde nun gefunden, daß N-substituierte 3-Azabicyclo [3.2.0]-heptan-Derivate der Formel I 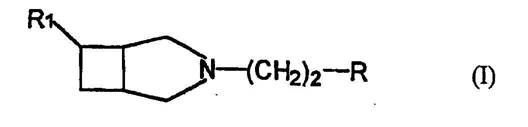 worin
- R¹: Naphthyl-, gegebenenfalls durch Fluor oder Chlor substituiert,
- R: 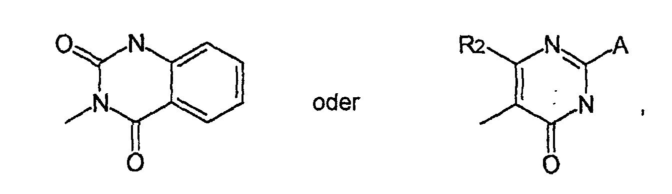
- R²: eine Hydroxy- oder Methylgruppe, und
- A: ein Wasserstoffatom, eine Hydroxy-, Amino-, Mercapto-, C₁-C₄-Alkylamino-, Di-C₁-C₄-alkylamino-, C₁-C₄-Alkylthio- oder C₁-C₄-Alkoxygruppe bedeutet
und deren Salze mit physiologisch verträglichen Säuren wertvolle pharmakologische Eigenschaften besitzen.

Bevorzugt sind insbesonders die Verbindungen, in denen sich das Ringsystem im rechten Teil des Moleküls von
2,4-(1H, 3H)-Chinazolindion oder 2-Methylamino-3, 6-dimethyl-4 (3H) pyrimidinon, ableitet.

Die erfindungsgemäßen Verbindungen der Formel I lassen sich herstellen, indem man eine Verbindung der Formel II

Nu―(CH₂)₂― R (II)

in der R die oben angegebenen Bedeutung hat und Nu eine nukleofuge Abgangsgruppe darstellt, mit einem 3-Azabicyclo[3 2.0]heptan-Derivat der Formel (III) 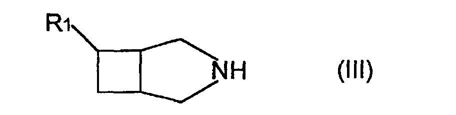 worin R¹ die oben angegebene Bedeutung hat, umsetzt und die so erhaltene Verbindung gegebenenfalls in das Säureadditionssalz einer physiologisch verträglichen Säure überführt.

Als nukleofuge Abgangsgruppe für Nu kommen vorzugsweise Halogenatome, insbesondere Brom oder Chlor, in Betracht.

Die Umsetzung erfolgt zweckmäßig in Gegenwart einer inerten Base, wie Triethylamin oder Kaliumcarbonat, als säurebindendes Mittel in einem inerten Lösungsmittel, wie einem cyclischen gesättigten Ether, insbesondere Tetrahydrofuran oder Dioxan, oder einem Ben_{zo}lkohlenwasserstoff, wie Toluol oder Xylol.

Die Umsetzung erfolgt in der Regel bei Temperaturen von 20 bis 150°C, insbesondere von 80 bis 140°C, und ist im allgemeinen innerhalb von 1 bis 10 Stunden beendet.

Die erfindungsgemäßen Verbindungen der Formel I können entweder durch Umkristallisation aus den üblichen organischen Lösungsmitteln, bevorzugt aus einem niederen Alkohol, wie Ethanol, umkristallisiert oder durch Säulenchromatographie gereinigt werden.

Racemate lassen sich in einfacher Weise durch klassische Spaltung mit optisch aktiven Carbonsäuren, z.B. Weinsäure-Derivaten, in einem inerten Lösungsmittel, z.B. niederen Alkoholen, in die Enantiomeren auftrennen.

Die freien 3-Azabicyclo[3.2.0]heptan-Derivate der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure oder Zitronensäure.

Die erfindungsgemäßen Verbindungen weisen wertvolle pharmakologische Eigenschaften auf. Sie können als Neuroleptika (insbesondere atypische), Antidepressiva, Sedativa, Hypnotika, ZNS-Protektiva oder Muskelrelaxantien insbesondere zur Behandlung von Psychosen Verwendung finden. Mehrere der genannten Wirkungsqualitäten können kombiniert bei einer erfindungsgemäßen Verbindung auftreten. Der pharmakologische Wirkungsnachweis erfolgt sowohl in vivo wie auch in vitro, wobei die Substanzcharakterisierung insbesondere durch die teilweise sehr hohe und selektive Affinität zu Rezeptor-Subtypen, vor allem Dopamin D₄-Rezeptoren, möglich ist.

Für die in vivo Charakterisierung wurden folgende Methoden herangezogen:

### a) Beeinflussung der Orientierungsmotilität

Mäuse zeigen in einer neuen Umgebung ein vermehrtes Explorationsverhalten, das sich in einer gesteigerten motorischen Aktivität äußert. Diese motorische Aktivität wird in Lichtschrankenkäfigen für die Zeit von 0 - 30 min nach Einsetzen der Tiere (NMRI-Mäuse, weibl.) in die Käfige gemessen. ED50: Dosis, die die motorische Aktivität im Vergleich zu Placebo-behandelten Kontrollen um 50 % reduziert.

### b) Apomorphin-Antagonismus

Weibliche NMRI-Mäuse erhalten 1,21 mg/kg Apomorphin s.c. Apomorphin führt in dieser Dosis zu einer motorischen Aktivierung, die sich, wenn man die Tiere in Maschendrahtkäfigen hält, in einem permanenten Klettern äußert. Das Klettern wird mit einem Score bewertet (alle 2 min während 30 min):
0: Tier hat vier Pfoten am Boden
1: Tier hat zwei Pfoten am Draht
2: Tier hat vier Pfoten am Draht (klettert).
Durch Vorbehandlung mit Antipsychotika ist das Kletterverhalten zu hemmen.
ED50: Dosis, die die Kletteraktivität der Tiere im Vergleich zu Placebo-behandelten Kontrollen um 50 % hemmt.

### c) Methamphetamin-Antagonismus

Weibliche NMRI-Mäuse erhalten 1 mg/kg Methamphetamin p.o. und werden nach 30 min in Lichtschrankenkäfige zur Messung der motorischen Aktivität eingesetzt (2 Tiere/Käfig, 4 Käfige/Dosis). Die Prüfsubstanzen werden 30 min vor Methamphetamin oral gegeben. Die Aktivitätssteigerung durch Methamphetamin wird für die Zeit 15 bis 60 min nach Einsetzen der Tiere in die Meßkäfige als Differenz von Methamphetaminkontrollen zu Placebokontrollen berechnet und gleich 100 % gesetzt. Die ED100 ist die Dosis der Prüfsubstanz, die die Aktivitätssteigerung vollständig aufhebt.

### d) L-5-HTP-Antagonismus

Weibliche Sprague-Dawley-Ratten erhalten L-5-HTP in einer Dosis von 316 mg/kg i.p. Die Tiere entwickeln darauf ein Erregungssyndrom, von dem Symptome
- for paw treading und
- tremor
mit Hilfe eines Scores (O = nicht vorhanden, 1 = mäßig, 2 = deutlich ausgeprägt) alle 10 min in der Zeit von 20 bis 60 min nach L-5-HTP-Gabe bewertet werden. Im Mittel wird nach L-5-HTP-Gabe ein Score von 17 erreicht. Die Prüfsubstanzen p.o. werden 60 min vor L-5-HTP gegeben. Als ED50 wird die Dosis errechnet, die im Mittel den Kontrollscore um 50 % vermindert.

Die aufgeführten Methoden sind geeignet, Substanzen als Antipsychotika zu charakterisieren; insbesondere die Hemmung von durch Methamphetamin induzierter motorischer Stimulierung gilt als prädiktiv für eine antipsychotische Wirkung. Mit der Hemmung des L-5-HTP-Syndroms kann eine Serotonin-antagonistische Wirkung aufgezeigt werden, eine Wirkungsqualität, wie sie für die sogenannten atypischen Neuroleptika charakteristisch ist.

In diesen Tests zeigen die neuen Verbindungen eine gute Wirkung.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen 0,1 und 10 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et. al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die als Ausgangsstoffe für die Synthese der neuen Verbindungen benötigten Substanzen der Formel II sind bekannt oder lassen sich gemäß den in der Literatur beschriebenen Herstellmethoden aus analogen Ausgangsmaterialien synthetisieren.

Die Substanzen der Formel III lassen sich herstellen, indem man ein Amin der Formel 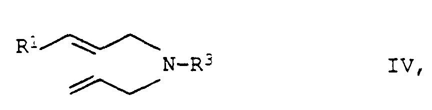 worin R¹ die oben angegebenen Bedeutungen besitzt und R³ Wasserstoff, Acetyl, Benzyl oder Trifluoracetyl bedeutet, photochemisch einer [2+2] Cycloaddition unterwirft und gegebenenfalls eine Acyl- oder Benzylgruppe abspaltet.

Die Photoreaktion gelingt gut in einem inerten Lösungsmittel, vorzugsweise Aceton, bei Temperaturen von 20 bis 80°C. Als Lichtquelle eignet sich besonders gut eine Quecksilberhochdrucklampe. Es ist gegebenenfalls vorteilhaft, die Photocycloaddition in einer Quarzapparatur unter einer Stickstoffatmosphäre gegebenenfalls unter Zusatz von etwa 1 Mol Salzsäure pro Mol Amin durchzuführen.

Die Photocycloaddition verläuft in den meisten Fällen hochdiastereoselektiv zu den bicyclischen Verbindungen III mit der exo-Konfiguration bezüglich R¹:

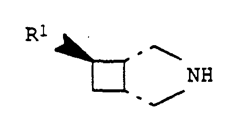

Durch Racematspaltung, z.B. mit optisch aktiven Weinsäure-Derivaten, lassen sich die beiden Enantiomeren rein isolieren.

Die Abspaltung einer Acylgruppe gelingt nach bekannten Methoden. Analoges gilt für die Entfernung einer Benzylgruppe.
Die Amine der Formel IV sind literaturbekannt oder lassen sich herstellen, indem man entweder einen Aldehyd R¹-CHO mit Vinylmagnesiumchlorid zum Allylalkohol V 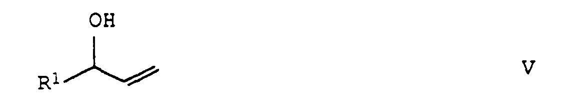 umsetzt, anschließend mit Chlorwasserstoff zum Allylchlorid VI 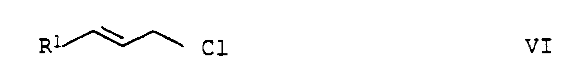 umlagert und zuletzt mit dem entsprechenden Allylamin VII 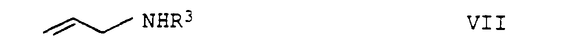 substituiert oder man unterzieht einen Zimtaldehyd VIII 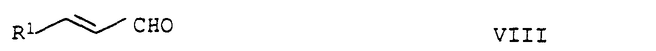 direkt der reduktiven Aminierung mit dem Allylamin VII mit R³ gleich Wasserstoff.

Die folgenden Beispiele dienen zur Erläuterung der Erfindung:
A Herstellung der Ausgangsmaterialien
   aa) 1-(1-Naphthyl)-allylalkohol
      In einen 2-1-Rührkolben wurden unter Stickstoff 277 ml (360 mM), einer 1,3 M Lösung von Vinylmagnesiumchlorid in Tetrahydrofuran gefüllt. Anschließend wurde unter Rühren und unter Stickstoff innerhalb von 60 min bei 30-35°C eine Lösung von 50 g (320 mM) 1-Naphthaldehyd gelöst in 250 ml Tetrahydrofuran zugegeben. Das Reaktionsgemisch wurde noch 4,5 h unter Stickstoff bei Raumtemperatur gerührt. Danach wurde unter Rühren und Kühlen mit Eis 90 ml gesättigte Ammoniumchlorid-Lösung zugesetzt, abgesaugt und der Filterrückstand dreimal mit 150 ml Tetrahydrofuran gewaschen. Die Filtrate wurden vereinigt, mit Natriumsulfat getrocknet und eingeengt. Man erhielt 58,3 g (99 %) rohes Produkt in Form eines braunen Öls.
   ab) 3-(1-Naphthyl)-allylchlorid
      58,3 g (317 mM) 1-(1-Naphthyl)-allylalkohol wurden unter Rühren in 400 ml Dichlormethan gelöst. Anschließend wurde bis zur Sättigung Chlorwasserstoff eingeleitet, wobei die Temperatur auf bis zu 37°C stieg. Es wurde 1 h nachgerührt. Nach dem Waschen mit 200 ml eiskaltem Wasser wurde die organische Phase über Natriumsulfat getrocknet und eingeengt. Man erhielt 59,2 g (92 %) bräunliche Festkörper.
   ac) N-Allyl-N-[3-(1-naphthyl)-allyl]-amin
      Zu 167 g (2,9 M) Allylamin wurden unter Rückfluß innerhalb von 1 h 59,2 g (0,29 M) 3-(1-Naphthyl)allylchlorid gelöst in 250 ml Toluol zugegeben. Die Mischung wurde 2 h bei Rückflußtemperatur nachgerührt. Anschließend wurde die Reaktionslösung eingeengt, der Rückstand in 250 ml Wasser aufgenommen und mit 50%iger Natronlauge auf pH 12 eingestellt. Die wäßrige Phase wurde mit Dichlormethan extrahiert, die organische Phase über Natriumsulfat getrocknet und eingeengt.
      Ausbeute: 67,6 g (97 %) dunkelbraunes Öl.
   ad) exo-6-(1-Naphthyl)-3-azabicyclo[3.2.0]heptan
      50,0 g (193 mM) N-Allyl-N-[3-(1-naphthyl)-allyl]ammoniumchlorid wurden in 1600 ml Aceton gelöst und mit 210 ml 10%iger Salzsäure versetzt. Die klare gelbe Lösung wurde unter Stickstoff mit einer 700 Watt Quecksilber-Hochdrucklampe in einer Quartzapparatur 4 h bei Raumtemperatur bestrahlt. Anschließend wurde die Reaktionslösung eingeengt, der Rückstand in Wasser aufgenommen und mit 50%iger Natronlauge auf pH 12 gestellt. Es wurde 30 min nachgerührt und zweimal mit tert.-Butyl-methylether extrahiert. Die vereinigten organischen Phasen wurden über Natriumsulfat getrocknet und eingeengt.
      Der dunkelbraune ölige Rückstand (43,2 g) wurde in 150 ml Isopropanol gelöst und mit 25,5 g (220 mM) Maleinsäure, gelöst in 220 ml Isopropanol, versetzt. Das ausgefallene Maleinat wurde abgesaugt, mit Isopropanol nachgewaschen und im Vakuumtrockenschrank bei 40°C über Nacht getrocknet. Ausbeute: 43,9 g (67 %) farbloses Pulver, Schmp.: 162-164°C (Maleinat) Analog lassen sich folgende Substanzen herstellen:
   ae) exo-6-(2-Naphthyl)-3-azabicyclo[3.2.0]heptan, Schmp.: 145-147°C (Maleinat)
   af) exo-6-(5-Chlor-1-naphthyl)-3-azabicyclo[3.2.0]heptan,
   ag) exo-6-(6-Chlor-2-naphthyl)-3-azabicyclo[3.2.0] heptan.
B Herstellung der Endprodukte

### Beispiel 1

3,6-Dimethyl-2-methylamino-5-[2-(exo-6-(2-naphthyl)-3-azabicyclo [3.2.0] heptan-3-yl)-ethyl)-3H-pyrimidin-4-on Dihydrochlorid

3,0 g (13,5 mM) exo-6-(2-Naphthyl)-3-azabicyclo[3.2.0]heptan in 70 ml Xylol wurden mit 2,9 g (13,5 mM) 3,6-Dimethyl-2-methylamino-5-(2-chlorethyl)-3H-pyrimidin-4-on sowie mit 2,8 g (20,3 mM) fein pulverisiertem Kaliumcarbonat und 0,5 g Kaliumjodid versetzt und unter gutem Rühren 12 h unter Rückfluß gekocht.

Nach dem Abkühlen engte man am Rotationsverdampfer ein und verteilte den Rückstand zwischen Methylenchlorid und Wasser. Die wäßrige Phase wurde zweimal mit Methylenchlorid nachextrahiert und darauf die organische Phase nach dem Trocknen mit Natriumsulfat eingeengt. Das Rohprodukt (5,0 g) reinigte man durch Säulenchromatographie (Kieselgel, Laufmittel Dichlormethan/Methanol 90/10) .

Die freie Base (2,8 g) nahm man in 150 ml Aceton auf und versetzte mit überschüssiger etherischer Salzsäure. Anschließend saugte man die Festkörper unter Stickstoff in der Kälte ab, wusch das Hydrochlorid mit wenig Aceton nach und trocknete das Salz auf der Nutsche unter Stickstoff. Man isolierte 3,2 g (46 %) Produkt x 2 HCl, Schmp. 225-228°C.

Analog lassen sich herstellen:
2. 3,6-Dimethyl-2-methylamino-5-[2-(exo-6-(1-naphthyl)-3-azabicyclo [3.2.0] heptan-3-yl> -ethyl] -3H-pyrimidin-4-on,
   Schmp.: 138-140°C (Dihydrochlorid),
3. 3, 6-Dimethyl-2-methylamino-5-[2-(exo-6-(5-chlor- 1-naphthyl)-3-azabicyclo [3.2.0]heptan-3-yl)-ethyl]-3H-pyrimidin-4-on,
4. 3,6-Dimethyl-2-methylamino-5-[2-(exo-6-(6-chlor-2-naphthyl)-3-azabicyclo[3.2.0]heptan-3-yl)-ethyl]-3H-pyrimidin-4-on,
   Schmp.: 260-262°C (Dihydrochlorid x 2H₂O),
6. 7-Methyl-6-[2-(exo-6-(2-naphthyl)-3-azabicyclo[3.2.0]-heptan-3-yl)-ethyl)-5H-thiazolo[3,2-a]pyrimidin-5-on,
7. 3-[2-(exo-6-(2-Napthyl)-3-azabicyclo[3.2.0]heptan-3-yl)-ethyl]- 1H,3H-chinazolin-2,4-dion,
   Schmp.: 161-164°C,
8. 3-[2-(exo-6-(1-Naphthyl)-3-azabicyclo[3.2.0]heptan-3-yl)-ethyl]-lH,3H-chinazolin-2,4-dion,
   Schmp.: ab 211°C Zersetzung,
9. 6-Fluor-3-[2-(exo-6-(1-naphthyl)-3-azabicyclo[3.2,0]heptan-3-yl)-ethyl]-lH,3H-chinazolin-2,4-dion,
   Schmp.: 196-198°C,
10. 3-[2-(exo-6-(5-chlor-2-naphthyl)-3-azabicyclo[3.2.0]heptan-3-yl)-ethyl] -1H,3H-chinazolin-2,4-dion,

## Patentansprüche

1. N-substituierte 3-Azabicyclo[3.2.O]heptan-Derivate der Formel (I) 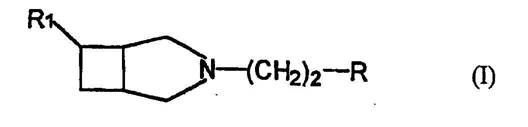 worin
R¹ Naphthyl-, gegebenenfalls durch Fluor oder Chlor substituiert,
R² eine Hydroxy- oder Methylgruppe,
R 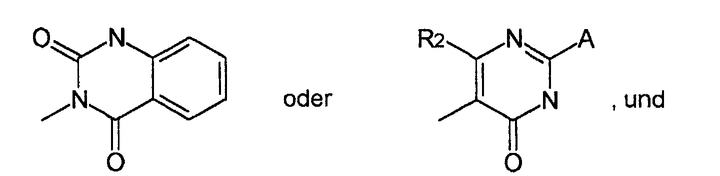
A ein Wasserstoffatom, eine Hydroxy-, Amino-, Mercapto-, C₁-C₄-Alkylamino-, Di-C₁-C₄-alkylamino-, C₁-C₄-Alkylthio- oder C₁-C₄-Alkoxygruppe
bedeutet,
und deren Salze mit physiologisch verträglichen Säuren.

2. Verbindungen der Formel (I) nach Anspruch 1 zur Verwendung bei der Bekämpfung von Krankheiten.

3. Verwendung einer Verbindung der Formel (1) nach Anspruch 1 zur Herstellung eines Arzneimittels zur Behandlung von Psychosen.

4. Verfahren zur Herstellung der Verbindungen der Formel (1) gemäß Anspruch 1, **dadurch gekennzeichnet, daß** man eine Verbindung der Formel (II)
Nu―(CH₂)₂―R (II)
in der R die oben angegebenen Bedeutung hat und Nu eine nukleofuge Abgangsgruppe darstellt, mit einem 3-Azabicyclo[3.2.0]heptan-Derivat der Formel (III) 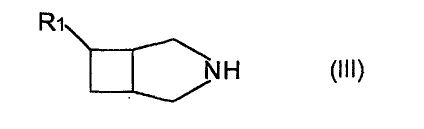 worin R¹ die oben angegebene Bedeutung hat, umsetzt und die so erhaltene Verbindung gegebenenfalls in das Säureadditionssalz einer physiologisch verträglichen Säure überführt.

## Claims

1. N-Substituted 3-azabicyclo[3.2.0]heptane derivatives of the formula (I) 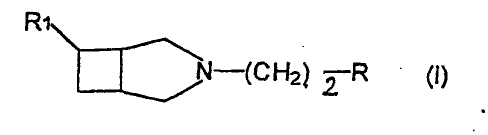 wherein
R¹ denotes naphthyl-, optionally substituted by fluorine or chlorine,
R² denotes a hydroxyl or methyl group,
R denotes 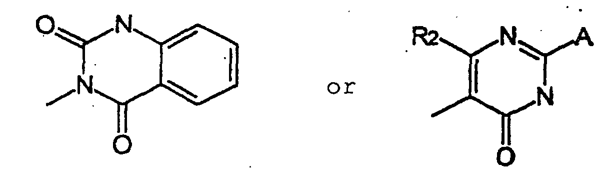 and
A denotes a hydrogen atom, a hydroxyl, amino, mercapto, C₁-C₄-alkylamino, di-C₁-C₄-alkylamino, C₁-C₄-alkylthio or C₁-C₄-alkoxy group,
and salts thereof with physiologically acceptable acids.

2. Compounds of the formula (I) according to claim 1 for use in combating diseases.

3. Use of a compound of the formula (I) according to claim 1 for the preparation of a medicament for treatment of psychoses.

4. Process for the preparation of the compounds of the formula (I) according to claim 1, **characterized in that** a compound of the formula (II)
Nu―(CH₂)₂―R (II)
in which R has the abovementioned meaning and Nu represents a nucleofugic leaving group, is reacted with a 3-azabicyclo [3.2.0] heptane derivative of the formula (III) 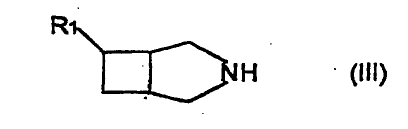 wherein R¹ has the abovementioned meaning, and the compound obtained in this way is optionally converted into the acid addition salt of a physiologically acceptable acid.

## Revendications

1. Dérivés de 3-azabicyclo[3.2.0]heptane N-substitués de formule (I) 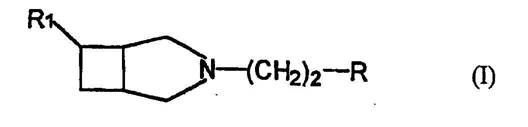 dans laquelle
R¹ représente un groupe naphtyle éventuellement substitué par du fluor ou du chlore,
R² représente un groupe hydroxy ou méthyle,
R représente 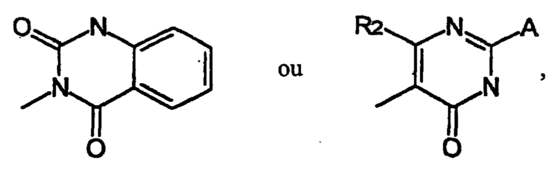 et
A représente un atome d'hydrogène ou un groupe hydroxy, amino, mercapto, alkylamino en C₁-C₄, di(alkyl en C₁-C₄)amino, alkylthio en C₁-C₄ ou alcoxy en C₁-C₄,
et leurs sels avec des acides physiologiquement acceptables.

2. Composés de formule (I) selon la revendication 1, à utiliser dans la lutte contre des maladies.

3. Utilisation d'un composé de formule (I) selon la revendication 1 pour la préparation d'un médicament destiné au traitement de psychoses.

4. Procédé de préparation des composés de formule (I) selon la revendication 1, **caractérisé en ce que** l'on fait réagir un composé de formule (II)
Nu-(CH₂)₂-R (II)
dans laquelle R a la signification indiquée ci-dessus et Nu représente un groupe partant nucléofuge, avec un dérivé de 3-azabicyclo[3.2.0]heptane de formule (III) 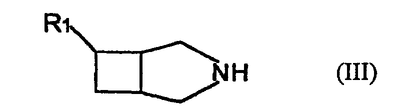 dans laquelle R¹ a la signification indiquée ci-dessus, et on transforme éventuellement le composé ainsi obtenu en le sel d'addition d'acide d'un acide physiologiquement acceptable.
